# EUROPÄISCHE PATENTSCHRIFT

(11) **EP 0 910 328 B1**
(45) Veröffentlichungstag und Bekanntmachung des Hinweises auf die Patenterteilung: **18.12.2002**
(21) Anmeldenummer: 97923976.1
(22) Anmeldetag: 22.05.1997
(51) Int. Cl.: A61K 7/06, A61K 7/04

(54) **WÄSSRIGE PERLGLANZKONZENTRATE**
AQUEOUS NACREOUS LUSTRE CONCENTRATE
CONCENTRES AQUEUX A LUSTRE NACRE

(30) Priorität: 30.05.1996 DE 19621681
(43) Veröffentlichungstag der Anmeldung: 28.04.1999
(73) Patentinhaber: Cognis Deutschland GmbH & Co. KG, 40589 Düsseldorf (DE)
(72) Erfinder: ANSMANN, Achim, D-40699 Erkrath (DE); KAWA, Rolf, D-40789 Monheim (DE); PODUBRIN, Stefan, D-45481 Mülheim (DE); WESTFECHTEL, Alfred, D-40724 Hilden (DE)
(86) Internationale Anmeldenummer: EP9702617
(87) Internationale Veröffentlichungsnummer: WO97046209

(56) Entgegenhaltungen:
- US-A- 4 078 147
- US-A- 4 438 096
- CHEMICAL ABSTRACTS, vol. 123, no. 16, 16.Oktober 1995 Columbus, Ohio, US; abstract no. 208463, SONE, HIROAKI ET AL: "Liquid detergent compositions with pearly gloss" XP002041409 & JP 07 082 140 A (KAO CORP, JAPAN)
- CHEMICAL ABSTRACTS, vol. 97, no. 8, 23.August 1982 Columbus, Ohio, US; abstract no. 60813, LION CORP., JAPAN: "Dicarboxylic acid esters as pearly substances for cosmetics" XP002041410 & JP 57 067 510 A (LION CORP., JAPAN)

## Beschreibung

### Gebiet der Erfindung

Die Erfindung betrifft wäßrige Perlglanzkonzentrate mit einem Gehalt an Estern mehrwertiger Hydroxycarbonsäuren, Emulgatoren und gegebenenfalls Polyolen, ein Verfahren zu ihrer Herstellung, ein weiteres Verfahren zur Herstellung von perlglänzenden oberflächenaktiven Zubereitungen unter Verwendung der Konzentrate sowie die Verwendung von Estem mehrwertiger Hydroxycarbonsäuren als Perlglanzwachse.

### Stand der Technik

Der weich schimmernde Glanz von Perlen hat auf den Menschen schon seit Jahrtausenden eine besondere Faszination ausgeübt. Es ist daher kein Wunder, daß die Hersteller von kosmetischen Zubereitungen versuchen, ihren Produkten ein attraktives, wertvolles und gehaltvolles Erscheinungsbild zu verleihen. Der erste seit dem Mittelalter in der Kosmetik eingesetzte Perlglanz war eine perlglänzende Paste aus natürlichen Fischschuppen. Zu Anfang dieses Jahrhunderts entdeckte man, daß Wismutoxidchloride ebenfalls in der Lage sind, Perlglanz zu erzeugen. Für die moderne Kosmetik sind hingegen Perlglanzwachse, insbesondere vom Typ der Glycolmono- und -difettsäureester von Bedeutung, die überwiegend zur Erzeugung von Perlglanz in Haarshampoos und Duschgelen eingesetzt werden. Eine Übersicht zu modernen, perlglänzenden Formulierungen findet sich von A.Ansmann und R.Kawa in **Parf.Kosm. 75, 578 (1994).**

Der Stand der Technik kennt eine Vielzahl von Formulierungen, die oberflächenaktiven Mitteln den gewünschten Perlglanz verleihen. So sind beispielsweise aus den beiden Deutschen Patentanmeldungen **DE-A1 38 43 572** und **DE-A1 41 03 551** (Henkel) Perlglanzkonzentrate in Form fließfähiger wäßriger Dispersionen bekannt, die 15 bis 40 Gew.-% perlglänzender Komponenten, 5 bis 55 Gew.-% Emulgatoren und 0,1 bis 5 bzw. 15 bis 40 Gew.-% Polyole enthalten. Bei den Perlglanzwachsen handelt es sich um acylierte Polyalkylenglycole, Monoalkanolamide, lineare, gesättigte Fettsäuren oder Ketosulfone. In den beiden Europäischen Patentschriften **EP-B1 0 181 773 und EP-B1 0 285 389** (Procter & Gamble) werden Shampoozusammensetzungen vorgeschlagen, die Tenside, nicht-flüchtige Silicone und Perlglanzwachse enthalten. Gegenstand der Europäischen Patentanmeldung **EP-A2 0 205 922** (Henkel) sind fließfähige Perlglanzkonzentrate, die 5 bis 15 Gew.-% acylierte Polyglycole, 1 bis 6 Gew.-% Fettsäuremonoethanolamide und 1 bis 5 Gew.-% nichtionische Emulgatoren enthalten. Gemäß der Lehre der Europäischen Patentschrift **EP-B1 0 569 843** (Hoechst) lassen sich nichtionische, fließfähige Perlglanzdispersionen auch erhalten, indem man Mischungen von 5 bis 30 Gew.-% acylierten Polyglycolen und 0,1 bis 20 Gew.-% ausgewählten nichtionischen Tensiden herstellt. Aus der Europäischen Patentanmeldung **EP-A2 0 581 193** (Hoechst) sind ferner fließfähige, konservierungsmittelfreie Perlglanzdispersionen bekannt, die acylierte Polyglycolether, Betaine, Aniontenside und Glycerin enthalten. In der Europäischen Patentanmeldung **EP-A1 0 684 302** (Th.Goldschmidt) wird die Verwendung von Polyglycerinestern als Kristallisationshilfsmittel für die Herstellung von Perlglanzkonzentraten vorgeschlagen. Schließlich offenbaren zwei Japanische Patentanmeldungen die Verwendung von Diestern der Bernsteinsäure - **JP 07 082140 A** (Kao Corp.) - und Malonsäure - **JP 57 067 510 A** (Lion Corp.) - als Perlglanzmittel in tensidhaltigen kosmetischen Zubereitungen.

Trotz der Vielzahl von Mitteln besteht im Markt ein ständiges Bedürfnis nach neuen Perlglanzwachsen, die beispielsweise im Gegensatz zu acylierten Polyglycolen keine Ethylenoxideinheiten aufweisen und sich gegenüber den Produkten des Stands der Technik auch bei verminderter Einsatzmenge durch einen brillanten Glanz auszeichnen, die die Mitverwendung kritischer Inhaltsstoffe wie beispielsweise von Siliconen zulassen, ohne daß die Stabilität der Formulierungen beeinträchtigt wird, gleichzeitig über Estergruppen verfügen, damit eine ausreichende biologische Abbaubarkeit gewährleistet ist und die insbesondere in konzentrierter Form noch leicht beweglich und damit handhabbar sind. Die Aufgabe der vorliegenden Erfindung hat somit darin bestanden, neue Perlglanzkonzentrate mit dem geschilderten komplexen Anforderungsprofil zur Verfügung zu stellen.

### Beschreibung der Erfindung

Gegenstand der Erfindung sind wäßrige Perlglanzkonzentrate, enthaltend - bezogen auf den nicht-wäßrigen Anteil -
(a) 1 bis 99,9 Gew.-% Ester von mehrwertigen Hydroxycarbonsäuren mit Fettalkoholen mit 6 bis 22 Kohlenstoffatomen,
(b) 0,1 bis 90 Gew.-% anionische, nichtionische, kationische, ampholytische und/oder zwitterionische Emulgatoren sowie
(c) 0 bis 40 Gew.-% Polyole,
mit der Maßgabe, daß sich die Mengenangaben zu 100 Gew.-% ergänzen.

Überraschenderweise wurde gefunden, daß Ester von mehrwertigen Hydroxycarbonsäuren mit Fettalkoholen ausgezeichnete perlglänzende Eigenschaften besitzen und sich gegenüber den Produkten des Stands der Technik durch eine höhere Brillanz bei geringerer Einsatzmenge, besondere Feinteiligkeit und Lagerstabilität auszeichnen. Die Perlglanzwachse sind leicht biologisch abbaubar, in konzentrierter Form dünnflüssig und erlauben auch die Einarbeitung von problematischen Inhaltsstoffen wie beispielsweise Siliconen in kosmetische Zubereitungen.

### Mehrwertige Hydroxycarbonsäureester

Bei den Perlglanzwachsen, die die Komponente (a) bilden, handelt es sich um bekannte Stoffe, die nach den einschlägigen Verfahren der präparativen organischen Chemie erhalten werden können. Üblicherweise erfolgt die Herstellung der Ester in an sich bekannter Weise durch basenkatalysierte Veresterung von Hydroxycarbonsäuren mit 4 bis 12 Kohlenstoffatomen, 2 bis 4 Carboxyl- und 1 bis 5 Hydroxylgruppen mit den Fettalkoholen. Als Säurekomponente kommen beispielsweise Äpfelsäure, Citronensäure und insbesondere Weinsäure und deren Mischungen in Betracht. Die Fettalkohole enthalten 6 bis 22, vorzugsweise 12 bis 18 und insbesondere 16 bis 18 Kohlenstoffatome in der Alkylkette. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Die Ester können als Voll- oder Partialester vorliegen, vorzugsweise werden Mono- und vor allem Diester der Hydroxycarbonsäuren eingesetzt. Typische Beispiele sind Weinsäuremono- und -dilaurylester, Weinsäuremono- und - dikokosalkylester, Weinsäuremono- und -dicetearylester, Citronensäuremono-, -di- und -trilaurylester, Citronensäuremono-, -di- und -trikokosalkylester sowie Citronensäuremono-, -di- und -tricetearylester. Die Einsatzmenge der Ester bezogen auf den nicht-wäßrigen Anteil der Konzentrate kann 1 bis 99,9 Gew.-% betragen.

### Emugatoren

Die erfindungsgemäßen Perlglanzkonzentrate können als Emulgatoren nichtionogene Tenside aus mindestens einer der folgenden Gruppen enthalten:
(b1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(b2) C_{12/18}-Fettsäuremono- und -diester von Anlagerungsprodukten von 1 bis 30 Mol Ethylenoxid an Glycerin;
(b3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(b4) Alkylmono- und -oligoglycoside mit 8 bis 22 Kohlenstoffatomen im Alkylrest und deren ethoxylierte Analoga;
(b5) Anlagerungsprodukte von 15 bis 60 Mol Ethylenoxid an Ricinusöl und/oder gehärtetes Ricinusöl;
(b6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(b7) Anlagerungsprodukte von 2 bis 15 Mol Ethylenoxid an Ricinusöl und/oder gehärtetes Ricinusöl;
(b8) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
(b9) Trialkylphosphate;
(b10) Wollwachsalkohole;
(b11) Polysiloxan-Polyalkyl-Polyether-Copolymere bzw. entsprechende Derivate;
(b12) Mischester aus Pentaerythrit, Fettsäuren, Citronensäure und Fettalkohol gemäß **DE-PS 11 65 574** sowie
(b13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Die erfindungsgemäßen Perlglanzkonzentrate können die Emulgatoren in Mengen von 0,1 bis 90 Gew.-%, bezogen auf den nicht-wäßrigen Anteil, enthalten.

### Polyole

Polyole, die im Sinne der Erfindung als Komponente (c) in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Die erfindungsgemäßen Perlglanzkonzentrate können die Polyole, vorzugsweise Glycerin, Propylenglycol, Butylenglycol, Hexylenglycol sowie Polyethylenglycole mit einem durchschnittlichen Molekulargewicht im Bereich von 100 bis 1.0000 in Mengen von 0,1 bis 40 Gew.-%, bezogen auf den nicht-wäßrigen Anteil, enthalten.

### Herstellverfahren

In einer bevorzugten Ausführungsform, die ebenfalls Gegenstand der Erfindung ist, erfolgt die Herstellung der Perlglanzkonzentrate, indem man eine Mischung aus den Komponenten (a), (b) und (c) herstellt, auf eine Temperatur erwärmt, die 1 bis 30°C oberhalb des Schmelzpunktes der Mischung liegt, mit der erforderlichen Menge Wasser etwa der gleichen Temperatur mischt und anschließend auf Raumtemperatur abkühlt. Ferner ist es möglich, eine konzentrierte wäßrige (Anion-)Tensidpaste vorzulegen, das Perlglanzwachs in der Wärme einzurühren und die Mischung anschließend mit weiterem Wasser auf die gewünschte Konzentration zu verdünnen oder das Vermischen in Gegenwart polymerer hydrophiler Verdickungsmittel, wie etwa Hydroxypropylcellulosen, Xanthan Gum oder Polymeren vom Carbomer-Typ durchzuführen.

### Gewerbliche Anwendbarkeit

Die erfindungsgemäßen Perlglanzkonzentrate eignen sich zur Einstellung einer Trübung in oberflächenaktiven Zubereitungen wie beispielsweise Haarshampoos oder manuellen Geschirrspülmitteln. Ein weiterer Gegenstand der Erfindung betrifft daher ein Verfahren zur Herstellung getrübter und perlglänzender flüssiger, wäßriger Zubereitungen wasserlöslicher grenzflächenaktiver Stoffe, bei dem man den klaren wäßrigen Zubereitungen bei 0 bis 40°C die Perlglanzkonzentrate in einer Menge von 0,5 bis 40, vorzugsweise 1 bis 20 Gew.-% der Zubereitung zusetzt und unter Rühren darin verteilt.

### Tenside

Die oberflächenaktiven Zubereitungen, die in der Regel einen nicht-wäßrigen Anteil im Bereich von 1 bis 50 und vorzugsweise 5 bis 35 Gew.-% aufweisen, können nichtionische, anionische, kationische und/oder amphotere Tenside enthalten, deren Anteil an den Mitteln üblicherweise etwa 50 bis 99 und vorzugsweise 70 bis 90 Gew.-% beträgt. Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofem die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, Alk(en)yloligoglykoside, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen, Die gleichen Tenside können auch unmittelbar zur Herstellung der Perlglanzkonzentrate eingesetzt werden.

### Hilfs- und Zusatzstoffe

Die oberflächenaktiven Zubereitungen, denen die erfindungsgemäßen Perlglanzkonzentrate zugesetzt werden, können weitere Hilfs- und Zusatzstoffe enthalten, wie beispielsweise Ölkörper, Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Adsorber, Farb- und Duftstoffe enthalten.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder aliphatische bzw. naphthenische Kohlenwasserstoffe in Betracht.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen in Betracht. Bevorzugt ist eine Kombination dieser Stoffe mit Alkyloligoglucosiden und/oder Fettsäure-N-methyl-glucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, kationische Stärke, Copolymere von Diallylammoniumsalzen und Acrylamiden, quatemierte VinylpyrrolidonNinylimidazol-Polymere wie z.B. Luviquat® (BASF AG, Ludwigshafen/ FRG), Kondensationsprodukte von Polyglycolen und Aminen, quatemierte Kollagenpolypeptide wie beispielsweise Lauryldimonium hydroxypropyl hydrolyzed collagen (Lamequat®L, Grünau GmbH), quaternierte Weizenpolypeptide, Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quatemiertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage. Im Sinne der Erfindung können neben den Hydroxycarbonsäureestern auch weitere bekannte **Perlglanzwachse** wie insbesondere Mono- und Difettsäureester von Polyalkylenglycolen oder Partialglyceride eingesetzt werden, Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen, Zur Verbesserung des Fließverhaltens können ferner **Hydrotrope** wie beispielsweise Ethanol, Isopropylalkohol, Propylenglycol oder Glucose eingesetzt werden. Als **Konservierungsmittel** eignen sich beispielsweise Phenoxyethanol, Formaldehydlösung, Parabene, Pentandiol oder Sorbinsäure. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

Ein letzter Gegenstand der Erfindung betrifft schließlich die Verwendung von Estern von mehrwertigen Hydroxycarbonsäuren mit Fettalkoholen mit 6 bis 22 Kohlenstoffatomen als Perlglanzwachse zur Herstellung von oberflächenaktiven Zubereitungen.

### Beispiele

Die erfindungsgemäßen Perlglanzkonzentrate R1 bis R4 sowie die Vergleichsmischungen R5-R7 wurden 14 Tage bei 40°C gelagert und die Viskosität nach der Brookfield-Methode in einem RVT-Viskosimeter (23°C, 10 Upm, Spindel 5) bestimmt. Anschließend wurden wäßrige Haarshampooformulierungen durch Vermischen der Einsatzstoffe bei 50°C zubereitet, die jeweils 2 g der Perlglanzkonzentrate R1 bis R7, 15 g Kokosfettalkohol+2EO-sulfat-Natriumsalz, 3 g Dimethylpolysiloxan, 5 g Kokosalkylglucosid und 1,5 g eines Esterquats (Wasser ad 100 Gew.-%) enthielten. Die Feinteiligkeit der Perlglanzkristalle in den Haarshampoos wurde unter dem Mikroskop visuell auf einer Skala von 1 = sehr feine Kristalle bis 5 = grobe Kristalle beurteilt. Die Beurteilung des Perlglanzes erfolgte ebenfalls auf einer Skala von 1 = brillant bis 5 = stumpf; die Trübung wurde visuell bestimmt und mit (+) = trüb oder (-) = trübungsfrei beurteilt. Die Zusammensetzungen und Ergebnisse sind in Tabelle 1 zusammengefaßt; alle Mengenangaben verstehen sich als Gew.-%

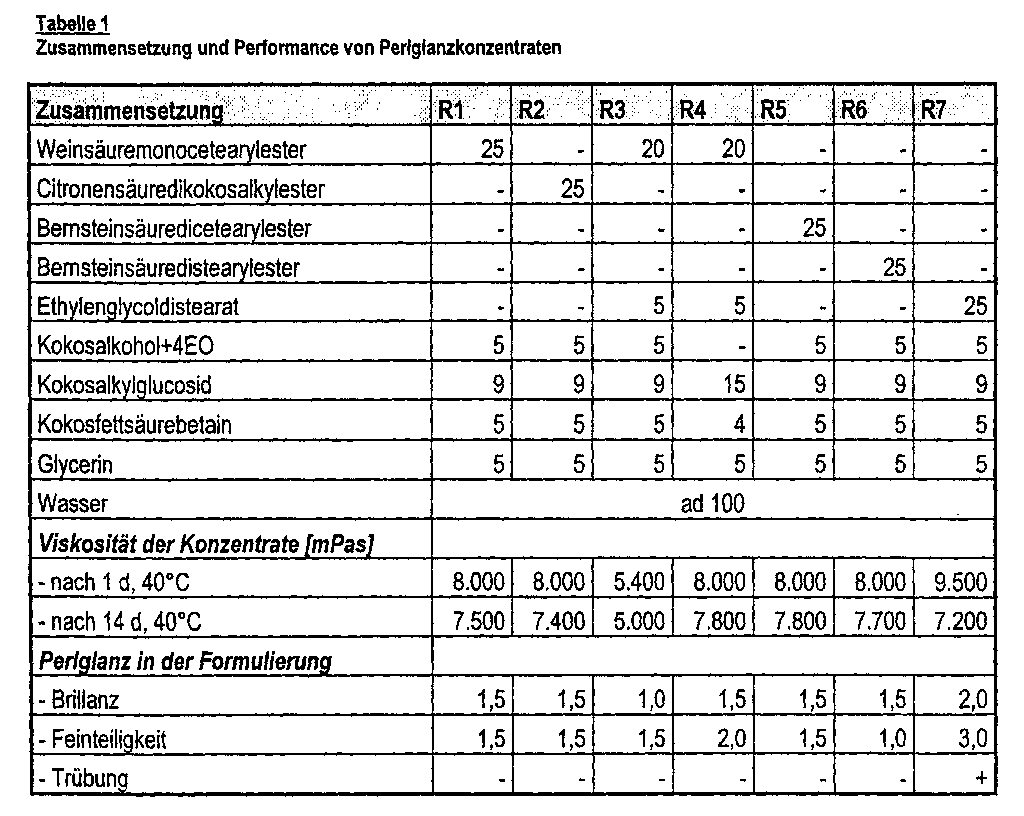

## Patentansprüche

1. Wäßrige Perlglanzkonzentrate, enthaltend - bezogen auf den nicht-wäßrigen Anteil -
(a) 1 bis 99,9 Gew.-% Ester von mehrwertigen Hydroxycarbonsäuren mit Fettalkoholen mit 6 bis 22 Kohlenstoffatomen,
(b) 0,1 bis 90 Gew.-% anionische, nichtionische, kationische, ampholytische und/oder zwitterionische Emulgatoren sowie
(c) 0 bis 40 Gew.-% Polyole,
mit der Maßgabe, daß sich die Mengenangaben zu 100 Gew.-% ergänzen.

2. Perlglanzkonzentrate nach Anspruch 1, **dadurch gekennzeichnet, daß** sie als Komponente (a) Ester der Weinsäure, Äpfelsäure, und/oder Citronensäure enthalten.

3. Perlglanzkonzentrate nach den Ansprüchen 1 und 2, **dadurch gekennzeichnet, daß** sie als Komponente (a) Ester von Fettalkoholen mit 12 bis 18 Kohlenstoffatomen enthalten.

4. Perlglanzkonzentrate nach den Ansprüchen 1 bis 3, **dadurch gekennzeichnet, daß** sie als Komponente (b) Emulgatoren enthalten, die ausgewählt sind aus der Gruppe, die gebildet wird von:
(b1) Anlagerungsprodukten von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(b2) C_{12/18}-Fettsäuremono- und -diestern von Anlagerungsprodukten von 1 bis 30 Mol Ethylenoxid an Glycerin;
(b3) Glycerinmono- und -diestern und Sorbitanmono- und -diestern von gesättigten und ungesättigten Fettsäuren mit 6 bis 22 Kohlenstoffatomen und deren Ethylenoxidanlagerungsprodukte;
(b4) Alkylmono- und -oligoglycosiden mit 8 bis 22 Kohlenstoffatomen im Alkylrest und deren ethoxylierten Analoga;
(b5) Anlagerungsprodukten von 15 bis 60 Mol Ethylenoxid an Ricinusöl und/oder gehärtetes Ricinusöl;
(b6) Polyolestern;
(b7) Anlagerungsprodukten von 2 bis 15 Mol Ethylenoxid an Ricinusöl und/oder gehärtetes Ricinusöl;
(b8) Partialestem auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkoholen sowie Polyglucosiden;
(b9) Trialkylphosphaten;
(b10) Wollwachsalkoholen;
(b11) Polysiloxan-Polyalkyl-Polyether-Copolymeren bzw. entsprechenden Derivaten;
(b12) Mischestern aus Pentaerythrit, Fettsäuren, Citronensäure und Fettalkoholen;
(b13) Polyalkylenglycolen.

5. Perlglanzkonzentrate nach den Ansprüchen 1 bis 3, **dadurch gekennzeichnet, daß** sie als Komponente (b) Emulgatoren vom Typ der zwitterionischen Tenside und/oder Esterquats enthalten.

6. Perlglanzkonzentrate nach den Ansprüchen 1 bis 5, **dadurch gekennzeichnet, daß** sie - bezogen auf den nicht-wäßrigen Anteil -
(a) mindestens 1 Gew.-% Ester von mehrwertigen Hydroxycarbonsäuren mit Fettalkoholen mit 6 bis 22 Kohlenstoffatomen,
(b) 0,1 bis 90 Gew.-% anionische, nichtionische, kationische, ampholytische und/oder zwitterionische Emulgatoren sowie
(c) 0,1 bis 40 Gew.-% Polyole ausgewählt aus der Gruppe, die gebildet wird von Glycerin, 1,2-Propylenglycol, Butylenglycol, Hexylenglycol und/ oder Polyethylenglycolen mit einem durchschnittlichen Molekulargewicht im Bereich von 100 bis 1.000 Dalton
enthalten.

7. Verfahren zur Herstellung von Perlglanzkonzentraten nach Anspruch 1, **dadurch gekennzeichnet, daß** man eine Mischung aus den Komponenten (a), (b) und gegebenenfalls (c) herstellt, auf eine Temperatur erwärmt, die 1 bis 30°C oberhalb des Schmelzpunktes der Mischung liegt, mit der erforderlichen Menge Wasser etwa der gleichen Temperatur mischt und anschließend auf Raumtemperatur abkühlt.

8. Verwendung von Estern von mehrwertigen Hydroxycarbonsäuren mit Fettalkoholen mit 6 bis 22 Kohlenstoffatomen als Perlglanzwachse zur Herstellung von oberflächenaktiven Zubereitungen.

## Claims

1. Aqueous pearlizing concentrates containing - based on the nonaqueous component -
(a) 1 to 99.9% by weight of esters of polybasic carboxylic acids with fatty alcohols containing 6 to 22 carbon atoms,
(b) 0.1 to 90% by weight of anionic, nonionic, cationic, ampholytic and/or zwitterionic emulsifiers and
(c) 0 to 40% by weight of polyois,
with the proviso that the quantities add up to 100% by weight.

2. Pearlizing concentrates as claimed in claim 1, **characterized in that** they contain esters of tartaric acid, malic acid and/or citric acid as component (a).

3. Pearlizing concentrates as claimed in claims 1 and 2, **characterized in that** they contain esters of fatty alcohols containing 12 to 18 carbon atoms as component (a).

4. Pearlizing concentrates as claimed in claims 1 to 3, **characterized in that** they contain as component (b) emulsifiers selected from the group consisting of:
(b1) products of the addition of 2 to 30 moles of ethylene oxide and/or 0 to 5 moles of propylene oxide onto linear fatty alcohols containing 8 to 22 carbon atoms, onto fatty acids containing 12 to 22 carbon atoms and onto alkylphenols containing 8 to 15 carbon atoms in the alkyl group;
(b2) C_{12/18} fatty acid monoesters and diesters of addition products of 1 to 30 moles of ethylene oxide to glycerol;
(b3) glycerol monoesters and diesters and sorbitan monoesters and diesters of saturated and unsaturated fatty acids containing 6 to 22 carbon atoms and ethylene oxide adducts thereof;
(b4) alkyl mono- and oligoglycosides containing 8 to 22 carbon atoms in the alkyl group and ethoxylated analogs thereof;
(b5) products of the addition of 15 to 60 moles of ethylene oxide onto castor oil and/or hydrogenated castor oil;
(b6) polyol esters;
(b7) products of the addition of 2 to 15 moles of ethylene oxide onto castor oil and/or hydrogenated castor oil;
(b8) partial esters based on linear, branched, unsaturated or saturated C_{12/22} fatty acids, ricinoleic acid and also 12-hydroxystearic acid and glycerol, polyglycerol, pentaerythritol, dipentaerythritol, sugar alcohols and polyglucosides;
(b9) trialkyl phosphates;
(b10) wool wax alcohols;
(b11) polysiloxane/polyalkyl polyether copolymers and corresponding derivatives thereof;
(b12) mixed ethers of pentaerythritol, fatty acids, citric acid and fatty alcohols;
(b13) polyalkylene glycols.

5. Pearlizing concentrates as claimed in claims 1 to 3, **characterized in that** they contain emulsifiers of the zwitterionic surfactant and/or esterquat type as component (b).

6. Pearlizing concentrates as claimed in claims 1 to 5, **characterized in that** they contain
(a) at least 1% by weight of esters of polybasic carboxylic acids with fatty alcohols containing 6 to 22 carbon atoms,
(b) 0.1 to 90% by weight of anionic, nonionic, cationic, ampholytic and/or zwitterionic emulsifiers and
(c) 0 to 40% by weight of polyols selected from the group consisting of glycerol, 1,2-propylene glycol, butylene glycol, hexylene glycol and/or polyethylene glycols with an average molecular weight of 100 to 1,000 dalton.

7. A process for the production of the pearlizing concentrates claimed in claim 1, **characterized in that** a mixture of components (a), (b) and (c) is prepared, heated to a temperature 1 to 30°C above the melting point of the mixture, mixed with the necessary quantity of water having substantially the same temperature and then cooled to room temperature.

8. The use of esters of polybasic carboxylic acids with fatty alcohols containing 6 to 22 carbon atoms as pearlizing waxes for the production of surface-active formulations.

## Revendications

1. Concentrés à éclat nacré aqueux contenant
- rapporté à la fraction non aqueuse -
(a) de 1 à 99,9 % en poids d'esters d'acide hydroxycarboxylique plurivalent avec des alcools gras ayant de 6 à 22 atomes de carbone,
(b) de 0,1 à 90 % en poids d'agents émulsionnants anioniques, non ioniques, cationiques, ampholytiques et/ou zwitterioniques ainsi que
(c) de 0 à 40 % en poids de polyols avec la précision que les données en quantités se complètent à 100 % en poids.

2. Condensés à éclat nacré selon la revendication 1,
**caractérisés en ce qu'**
ils contiennent comme composant (a) des esters d'acide tartrique, d'acide malique et/ou d'acide citrique.

3. Concentrés à éclat nacré selon les revendications 1 et 2,
**caractérisés en ce qu'**
ils contiennent comme composant (a) des esters d'alcool gras ayant de 12 à 18 atomes de carbone.

4. Concentrés à éclat nacré selon les revendications 1 à 3,
**caractérisés en ce qu'**
ils contiennent comme composant (b) des agents émulsionnants qui sont choisis dans le groupe formé de :
(b1) produits d'addition de 2 à 30 moles d'oxyde d'éthylène et/ou de 0 à 5 moles d'oxyde de propylène sur des alcools gras linéaires ayant de 8 à 22 atomes de carbone, sur des acides gras ayant de 12 à 22 atomes de carbone et sur des alkylphénols ayant de 8 à 15 atomes de carbone dans le groupe alkyle,
(b2) des mono- et des diesters d'acide gras en C₁₂/C₁₈ de produits d'addition de 1 à 30 moles d'oxyde d'éthylène sur le glycérol,
(b3) des mono- et des diesters de glycérol et des mono- et des diesters de sorbitane d'acides gras saturés et non saturé ayant 6 à 22 atomes de carbone, et leurs produits d'addition en oxydes d'éthylène,
(b4) des alkylmono- et oligo glycosides ayant de 8 à 22 atomes de carbone dans le reste alkyle et leurs analogues éthoxylés,
(b5) des produits d'addition de 15 à 60 moles d'oxyde d'éthylène sur de l'huile de ricin et/ou de l'huile de ricin durcie,
(b6) des esters de polyol,
(b7) des produits d'addition de 2 à 15 moles d'oxyde d'éthylène sur de l'huile de ricin et/ou de l'huile de ricin durcie,
(b8) des esters partiels à base d'acides gras linéaires ramifiés, non saturés ou saturés en C₁₂/C₂₂, d'acide ricinolique ainsi que de l'acide 12- hydroxystéarique et de glycérol, de polyglycérol de pentaérythritol, de dipentaérythritol, d'alcools de sucre ainsi que de polyglucosides,
(b9) des phosphates de trialkyle,
(b10) des alcools de cire de laine,
(b11) des copolymères polysiloxane-polyalkyl-polyéther ou les dérivés correspondants,
(b12) des esters mixtes à base de pentaérythritol, d'acides gras, d'acide citrique et d'alcools gras,
(b13) de polyalkylèneglycol.

5. Concentrés à éclat nacré selon les revendications 1 à 3,
**caractérisés en ce qu'**
ils renferment comme composant (b) des agents émulsionnants du type des agents tensioactifs zwitterioniques et/ou des esterquats.

6. Concentrés à éclat nacré selon les revendications 1 à 5,
**caractérisés en ce qu'**
ils contiennent - rapporté à la fraction non aqueuse -
(a) au moins 1 % en poids d'ester d'acide hydroxycarboxylique plurifonctionnel avec des alcools gras ayant de 6 à 22 atomes de carbone,
(b) de 0,1 à 90 % en poids d'agents émulsionnants anioniques, non ioniques, cationiques, ampholytiques, et/ou zwitterioniques ainsi que
(c) de 0,1 à 40 % en poids de polyols choisis dans le groupe formé du glycérol, du 1,2-propylèneglycol, du butylèneglycol, de l'hexylèneglycol, et/ou des polyéthylèneglycol ayant un poids moléculaire moyen dans la zone de 100 à 1000 daltons.

7. Procédé de préparation de concentrés à éclat nacré selon la revendication 1,
**caractérisé en ce qu'**
on prépare un mélange à base des composants (a), (b) et éventuellement (c), on chauffe à une température qui se situe de 1 à 30°C au-dessus du point de fusion du mélange, on mélange avec la quantité nécessaire d'eau d'environ la même température, et ensuite on refroidit à la température ambiante.

8. Utilisation d'esters d'acides hydroxycarboxyliques plurifonctionnels avec des alcools gras ayant de 6 à 22 atomes de carbone, en tant que cires à éclat nacré pour la production de préparations actives sur la tension superficielle.
